# FASCICULE DE BREVET EUROPEEN

(11) **EP 1 552 806 B1**
(45) Date de publication et mention de la délivrance du brevet: **10.09.2008**
(21) Numéro de dépôt: 05300007.1
(22) Date de dépôt: 06.01.2005
(51) Int. Cl.: A61K 8/81, A61K 8/90, A61Q 1/10

(54) **Composition de maquillage des fibres kératiniques à tenue dans le temps prolongée**
Langhaftende Schinkzusammensetzung für keratinische Fasern
Long lasting make-up composition for keratin fibers

(30) Priorité: 06.01.2004 FR 0450015
(43) Date de publication de la demande: 13.07.2005
(73) Titulaire: L'ORÉAL, 75008 Paris (FR)
(72) Inventeur: Pays, Karl, 94410 Saint-Maurice (FR)
(74) Mandataire: Le Coupanec, Pascale A.M.P.

(56) Documents cités:
- EP-A- 1 023 890
- EP-A- 1 249 223
- EP-A- 1 356 799

## Description

La présente invention se rapporte au maquillage de matières kératiniques notamment de fibres kératiniques, comme les cils, les sourcils et les cheveux, et plus particulièrement au maquillage des cils.

La composition selon l'invention peut se présenter sous la forme d'un mascara, d'un produit pour les sourcils, d'un eye-liner ou d'un produit de maquillage des cheveux. Plus spécialement, l'invention porte sur un mascara. Il peut notamment s'agir d'une composition de maquillage, d'une composition à appliquer sur ou sous un maquillage, dites encore respectivement « top-coat » ou « base-coat », ou bien encore d'une composition de traitement des cils.

D'une manière générale, les compositions de maquillage de fibres kératiniques et notamment de cils sont constituées d'au moins une cire ou d'un mélange de cires dispersé dans une phase liquide. C'est principalement à travers la quantité de cire et des autres ingrédients non volatils que sont ajustées les spécificités d'application recherchées pour les compositions, comme par exemple leur fluidité, leur pouvoir couvrant, leur pouvoir recourbant, leur pouvoir épaississant (encore appelé pouvoir chargeant ou maquillant), et leur tenue dans le temps. Les documents, EP-A-1249223 et EP-A-1356799 décrivent des compositions de mascara contenant des polymères filmogènes et semicristallins.

La présente invention a précisément pour objectif de proposer une composition cosmétique de soin et/ou de maquillage des fibres kératiniques possédant des propriétés améliorées en terme de tenue dans le temps.

En effet, les compositions de maquillage des fibres kératiniques et en particulier des cils, actuellement disponibles ne permettent pas d'obtenir un film possédant une résistance significative au frottement. Ces films ont généralement tendance à se désagréger au moins en partie, notamment en s'effritant ou par étalement. L'effritement partiel du film se traduit par une perte sensible de l'intensité de la couleur de maquillage et seule une nouvelle application de la composition sur le support déjà maquillé permet de suppléer à cette perte d'intensité. Quant à l'étalement du film, il se traduit en outre par le formation d'une auréole dans la proximité de la zone maquillée, qui, pour des raisons esthétiques évidentes, est totalement indésirable.

De manière inattendue, les inventeurs ont constaté que ce défaut de tenue dans le temps pouvait être surmonté efficacement à l'aide d'une nouvelle composition de soin et/ou de maquillage des fibres kératiniques.

De façon plus précise, la présente invention a pour objet une composition cosmétique de soin et/ou maquillage de fibres kératiniques comprenant dans un milieu solvant au moins :
(i) un polymère filmogène,
(ii) un polymère à groupement(s) styrénique(s) distinct du composé (i) et,
(iii) au moins un polymère soluble dans le milieu solvant, ayant au moins une partie cristallisable, et distinct du composé (i), ledit polymère étant présent en une teneur allant de 0,1 à 20% en poids par rapport au poids total de la composition.

Les inventeurs ont ainsi constaté que l'utilisation combinée de ces trois composés spécifiques au sein d'une formulation cosmétique notamment de type mascara permettrait de prolonger significativement la tenue dans le temps du film de maquillage correspondant, et ce jusqu'à au moins deux jours. Comme il ressort des exemples ci-après, la résistance à l'eau de ce film s'en trouve en particulier, significativement renforcée. Enfin, cette amélioration est obtenue conjointement avec un aspect plus lisse du film appliqué, résultant vraisemblablement d'une meilleure dispersion des particules de cires et des pigments au sein de la formulation selon l'invention.

La présente invention se rapporte en outre à un procédé de maquillage des fibres kératiniques, selon lequel on applique sur lesdites fibres kératiniques, notamment les cils, une composition telle que définie précédemment.

### TENUE DANS LE TEMPS

Comme précisé précédemment, les compositions conformes à l'invention possèdent une tenue dans le temps significativement améliorée par rapport à celle manifestée par des compositions de maquillage notamment de type mascara classiques.

Cette tenue dans le temps peut être appréciée selon différents protocoles. Dans le cas de la présente invention, le test plus particulièrement retenu vise à estimer la tenue d'un film aux frottements à l'eau.

Dans le premier test, des éprouvettes standardisées de faux cils, constitués à partir de cheveux caucasiens, sont maquillées selon le protocole suivant : 3 fois 10 passages de brosse avec 2 minutes de séchage entre chaque série puis séchage pendant une heure à température ambiante (25 °C).

Les éprouvettes sont ensuite pincées avec un coton disque imbibé d'eau.

L'évaluation de la tenue consiste à compter le nombre de cotons qu'il faut pour désagréger le film de mascara. Le début de la désagrégation correspond à l'apparition de traces de mascara sur le coton. La précision de cette méthode peut être estimée avec un delta d'erreur de +/- 2 cotons.

### MILIEU SOLVANT

Selon un mode de réalisation particulier, la présente invention concerne plus particulièrement le domaine des compositions de maquillage de fibres kératiniques à faible teneur en eau et/ou en solvants hydrosolubles, dits « mascaras waterproof », se présentant sous forme de dispersion de cire(s) dans des solvants non aqueux.

Ainsi, selon une variante particulière, la composition selon l'invention comprend un milieu solvant non aqueux.

Ce milieu est susceptible de former une phase continue et contient, comme son nom l'indique, au moins un solvant, notamment non aqueux qui est généralement un composé volatil, non soluble dans l'eau et liquide à température ambiante et pression atmosphérique.

Par « composé volatil », on entend, au sens de l'invention, tout composé (ou milieu non aqueux) susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par opposition, on entend par « composé non volatil », un composé restant sur la peau ou la fibre kératinique à température ambiante et pression atmosphérique, au moins plusieurs heures et ayant notamment une pression de vapeur inférieure à 10⁻³ mm de Hg (0,13Pa).

La teneur en composé volatil, non soluble dans l'eau et liquide à température ambiante est généralement de 5 à 95 %, notamment de 10 à 80 %, et en particulier de 30 à 70 % en poids par rapport au poids total de la composition.

Le composé volatil, non soluble dans l'eau et liquide à température ambiante est en particulier une huile ou un solvant organique cosmétiquement acceptable. Par l'expression « cosmétiquement acceptable », on entend un composé dont l'utilisation est compatible avec une application sur les fibres kératiniques et sur la peau.

Bien entendu, le milieu solvant de la composition selon l'invention peut comprendre un mélange de tels composés.

Les huiles volatiles peuvent être des huiles hydrocarbonées, des huiles siliconées, des huiles fluorées ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux « d'Isopars^{®} » ou de « Permetyls^{®} », les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination « Shell Solt^{®} » par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes (6.10⁻⁶ m²/s), et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants organiques volatils notamment fluorés tels que le nonafluorométhoxy butane ou le perfluorométhylcyclopentane.

Selon un mode de réalisation particulier des compositions selon l'invention, le composé volatil, non soluble dans l'eau et liquide à température ambiante est choisi parmi les huiles volatiles hydrocarbonées ayant de 8 à 16 atomes de carbone et leurs mélanges.

Le milieu solvant non aqueux peut également comprendre au moins un composé non volatil, non soluble dans l'eau et liquide à température ambiante, notamment au moins une huile non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STEARINERIES DUBOIS ou ceux vendus sous les dénominations de « Miglyol 810^{®} », « 812^{®} » et « 818^{®} » par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéaryle, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alkoxy, pendants et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans la composition de l'invention sont notamment des huiles fluorosiliconées, des polyéthers fluorés, des silicones fluorées telles que décrites dans le document EP-A-847 752.

Selon un mode de réalisation particulier, les compositions selon l'invention peuvent comprendre de l'eau et/ou au moins un solvant hydrosoluble.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention sont généralement en outre, volatils.

Parmi les solvants hydrosolubles pouvant être utilisés dans les compositions selon l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄.

L'eau et/ou le(s) solvant(s) hydrosoluble(s) peuvent être introduits en tant que tels dans la formulation selon l'invention ou y être incorporés par le biais, d'un ou plusieurs ingrédients constituant ladite composition. Ainsi de l'eau peut être notamment introduite dans la composition par le biais de l'introduction d'une dispersion aqueuse de particules de polymère utile par exemple à titre d'agent filmogène.

S'agissant de mascaras waterproof, la teneur en eau et/ou solvant(s) hydrosoluble(s) dans lesdites compositions est toutefois inférieure ou égale à 20 % et est généralement supérieure ou égale à 0,5 % en poids par rapport au poids total de la composition.

La teneur en eau et/ou en solvant(s) hydrosoluble(s) dans les compositions de l'invention peut en particulier varier de 1 à 18 % et plus particulièrement de 2 à 15 % en poids par rapport au poids total de la composition.

### POLYMERE FILMOGENE

La composition selon l'invention comprend au moins un polymère filmogène.

Dans la présente invention, on entend par « polymère filmogène », un polymère apte à former à lui seul ou en présence d'un agent auxiliaire de filmification, un film continu et adhérent sur un support, notamment sur les matières kératiniques.

D'une manière générale, le polymère filmogène peut être présent dans la composition selon l'invention en une teneur en matières sèches allant de 0,1 % à 55 % en poids par rapport au poids total de la composition, en particulier de 0,5 % à 40 % en poids, et plus particulièrement de 1 % à 30 % en poids.

En fait, cette quantité est susceptible de varier de manière significative selon la nature chimique du polymère filmogène retenu. L'élément déterminant est que cette quantité soit au moins suffisante pour attribuer la tenue prolongée attendue, à travers notamment une résistance optimisée aux frottements. Au-delà de cette quantité minimale, un excès en polymère filmogène peut ne pas être nécessaire. Toutefois, dans la mesure où il ne s'avère pas préjudiciable aux qualités attendues, rien ne s'oppose à la mise en oeuvre de ce polymère à une quantité supérieure à la quantité dite minimale.

Le polymère filmogène peut être présent dans la composition sous une forme liposoluble, hydrosoluble ou sous la forme de particules en dispersion dans un milieu aqueux ou un milieu solvant non aqueux.

### FORME LIPOSOLUBLE

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 24 atomes de carbone, lié au carbonyle du groupe ester) et d'au moins un autre monomère qui peut être un ester vinylique (différent de l'ester vinylique déjà présent), un alkylvinyléther (dont le groupe alkyle comporte de 2 à 24 atomes de carbone), ou un ester allylique ou méthallylique (ayant un radical hydrocarboné saturé, linéaire ou ramifié, de 1 à 24 atomes de carbone, lié au carbonyle du groupe ester). Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

A titre illustratif, on peut citer les copolymères suivants : acétate de vinyle/laurate de vinyle, propionate de vinyle/laurate d'allyle, propionate de vinyle/laurate de vinyle, et diméthyl-2, 2 pentanoate d'allyle/laurate de vinyle.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 2 à 24 atomes de carbone.

Comme exemples d'homopolymères liposolubles, on peut citer notamment: les polylaurate de vinyle et le poly(méth)acrylates de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2 232 303 ; ils peuvent avoir un poids moléculaire moyen en poids allant de 2 000 à 500 000 et en particulier de 4 000 à 200 000.

Comme polymères filmogènes liposolubles utilisables dans l'invention, on peut également citer les polyalkylènes et notamment les copolymères d'alcènes en C₂ à C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et en particulier en C₃ à C₂₀. A titre d'exemple de copolymères de VP utilisables dans l'invention, on peut citer les copolymères de VP/acétate vinyle, VP/méthacrylate d'éthyle, la polyvinylpyrrolidone (PVP) butylée, VP/méthacrylate d'éthyle/acide méthacrylique, VP/eicosène, VP/hexadécène, VP/triacontène, VP/styrène et VP/acide acrylique/méthacrylate de lauryle.

### FORME DISPERSEE

Le polymère filmogène peut être également présent dans la composition sous la forme de particules en dispersion dans une phase aqueuse, connue généralement sous le nom de latex ou pseudolatex, dans une phase solvant non aqueuse ou phase grasse liquide. Les techniques de préparation de ces dispersions sont bien connues de l'homme du métier.

### a) Dispersion aqueuse

Comme dispersion aqueuse de polymère filmogène, on peut utiliser les dispersions acryliques vendues sous les dénominations « Neocryl XK-90^{®} », « Neocryl A-1070^{®} », « Neocryl A-1090^{®} », « Neocryl BT-62^{®} », « Neocryl A-1079^{®} » et « Neocryl A-523^{®} » par la société AVECIA-NEORESINS, « Dow Latex 432^{®} » par la société DOW CHEMICAL, « Daitosol 5000 AD^{®} » par la société DAITO KASEY KOGYO; ou encore les dispersions aqueuses de polyuréthane vendues sous les dénominations «Neorez R-981^{®}» et «Neorez R-974^{®}» par la société AVECIA-NEORESINS, les «Avalure UR-405^{®}», «Avalure UR-410^{®}», «Avalure UR-425^{®}», «Avalure UR-450^{®}», «Sancure 875^{®}», «Sancure 861^{®}», «Sancure 878^{®}» et «Sancure 2060^{®}» par la société GOODRICH, «Impranil 85^{®}» par la société BAYER, « Aquamere H-1511^{®} » par la société HYDROMER ; les sulfopolyesters vendus sous le nom de marque « Eastman AQ^{®} » par la société EASTMAN CHEMICAL PRODUCTS, les dispersions vinyliques comme le « Mexomère PAM » par la société CHIMEX.

Il s'agit plus particulièrement d'homopolymères ou de copolymères blocs greffés ou séquencés incorporant au moins un bloc de polymère styrénique. En particulier, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène.

### b) Dispersion non aqueuse

Le polymère utilisé selon cette variante peut être de toute nature. On peut ainsi employer un polymère radicalaire, un polycondensat, voire un polymère d'origine naturelle et leurs mélanges. Le polymère peut être choisi par l'homme du métier en fonction de ses propriétés.

L'un des avantages de ce type de dispersion de polymère dans une composition de l'invention est la possibilité de faire varier la température de transition vitreuse (Tg) du polymère ou du système polymérique (polymère plus additif du type plastifiant), et de passer ainsi d'un polymère mou à un polymère plus ou moins dur, permettant de régler les propriétés mécaniques du film obtenu avec la composition de l'invention.

Les polymères utilisables dans la composition de l'invention ont de préférence un poids moléculaire moyen en poids allant de l'ordre de 2 000 à 10 000 000. Le polymère peut avoir une température de transition vitreuse allant de -100 °C à 300 °C, et mieux de -10 °C à 50 °C.

Il est possible d'utiliser des polymères filmifiables, de préférence ayant une température de transition vitreuse basse, inférieure ou égale à la température de la peau, et notamment inférieure ou égale à environ 40 °C. On entend par « polymère filmifiable » un polymère apte à former à lui seul, ou en présence d'un agent plastifiant, un film isolable. On obtient ainsi une dispersion qui peut filmifier lorsqu'elle est appliquée sur un support, ce qui n'est pas le cas lorsque l'on utilise des dispersions de pigments minéraux selon l'art antérieur.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à environ 40 °C et notamment allant de -10 °C à 30 °C, et leurs mélanges.

Parmi les polymères non filmifiables, on peut citer les homopolymères ou copolymères radicalaires, vinyliques ou acryliques, ayant de préférence une Tg supérieure à environ 40 °C, et notamment allant de 45 °C à 100 °C, et leurs mélanges. Le polymère non filmifiable permet, en association avec la phase grasse liquide, de former un dépôt continu et homogène sur les cils.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelés les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.

Parmi les (méth)acrylates d'alkyle, on peut citer le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle.

Parmi les (méth)acrylates d'hydroxyalkyle, on peut citer le (méth)acrylate d'hydroxyéthyle, le (méth)acrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth)acrylate d'alkyle, notamment d'alkyle en C₁-C₄. Plus préférentiellement, on peut utiliser les acrylates ou méthacrylates de méthyle copolymérisés avec l'acide acrylique.

Comme amides des monomères acides, on peut utiliser les (méth)acrylamides, et notamment les N-alkyl (méth)acrylamides, en particulier d'alkyl en C₂-C₁₂ tels que le N-éthyl acrylamide, N-t-butyl acrylamide, le N-octyl acrylamide ; les N, N-dialkyl (C₁-C₄) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, le vinylamine, le vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :
- la N-vinylpyrrolidone ; la vinylcaprolactame ; les vinyl N-alkyl(C₁-C₆) pyrroles ; les vinyl-oxazoles ; les vinyl-thiazoles ; les vinylpyrimidines ; les vinylimidazoles ;
- les oléfines tels que l'éthylène, le propylène, le butylène, l'isopropène, le butadiène.

Le polymère vinylique peut être réticulé à l'aide de monomère difonctionnel, notamment comprenant au moins deux insaturations éthyléniques, tel que le diméthacrylate d'éthylène glycol, le phtalate de diallyle.

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse alkyle ; polymères ou copolymères acryliques et/ou vinyliques ; polyacrylamides ; polymères siliconés ; polymères fluorés et leurs mélanges.

Avec une telle dispersion de particules de polymère, il est possible de calibrer à volonté la taille des particules de polymère, et de moduler leur « polydispersité » en taille lors de la synthèse. Il est ainsi possible d'obtenir des particules de très petite taille, qui sont invisibles à l'oeil nu lorsqu'elles sont dans la composition. Les nanoparticules peuvent en particulier posséder une taille allant de 5 à 600 nm, étant donné qu'au-delà d'environ 600 nm, les dispersions de particules deviennent beaucoup moins stables, et de préférence de 50 nm à 250 nm.

Le polymère dispersé est généralement utilisé en une quantité efficace pour obtenir un film déposé sur les fibres kératiniques résistant aux frottements éventuellement en présence d'eau et/ou de sébum et/ou de transpiration.

En pratique, le polymère dispersé dans une phase grasse liquide peut être présent en une teneur allant de 2 % à 50 % en poids, par rapport au poids total de la composition, de préférence de 4 % à 40 % en poids, et mieux de 5 % à 30 % en poids.

Par « phase grasse liquide », on entend tout milieu non aqueux liquide à température ambiante (25 °C) et pression atmosphérique. Cette phase grasse peut contenir une phase grasse liquide volatile et/ou une phase grasse liquide non volatile contenant respectivement une ou plusieurs huiles liquides.

Par « phase grasse liquide », on entend tout milieu non aqueux susceptible de s'évaporer de la peau en moins d'une heure. Cette phase volatile comporte notamment des huiles ayant une pression de vapeur, à température ambiante et pression atmosphérique allant de 10⁻³ à 300 mm de Hg (0,13 Pa à 40 000 Pa).

La phase grasse liquide dans laquelle est dispersé le polymère, peut être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

La phase grasse liquide totale de la composition peut représenter de 5 % à 98 % en poids, par rapport au poids total de la composition et de préférence de 20 à 85 % en poids.

Comme phase grasse liquide utilisable dans l'invention, on peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxane (PDMS), éventuellement phénylées telles que les phényltriméthicones, ou éventuellement substituées par des groupements aliphatiques et/ou aromatiques, éventuellement fluorés, ou par des groupements fonctionnels tels que des groupements d'hydroxyle, thiol et/ou amine ; les polysiloxanes modifiées par des acides gras, des alcools gras ou des polyoxyalkylènes, les silicones fluorées, les huiles perfluorées.

Avantageusement, on peut utiliser une ou plusieurs huiles volatiles à température ambiante. Ces huiles volatiles sont favorables à l'obtention d'un film à propriétés « sans transfert » total, à savoir résistant totalement aux frottements. Après évaporation de ces huiles, on obtient un dépôt filmogène souple, non collant. Ces huiles volatiles facilitent, en outre, l'application de la composition sur les fibres kératiniques comme les cils.

Ces huiles volatiles peuvent être des huiles hydrocarbonées, des huiles siliconées comportant éventuellement des groupements alkyle ou alkoxy pendants et/ou en bout de chaîne siliconée.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. On peut citer notamment l'octaméthylcyclotétrasiloxane, le décaméthyl-cyclopentasiloxane, l'hexadécaméthylcyclohexasiloxane, l'heptaméthylhexyltrisiloxane, l'heptaméthyloctyltrisiloxane.

Comme huile hydrocarbonée volatile, on peut citer les isoparaffines en C₈-C₁₆ telles que les ISOPARs^{®}, les PERMETYLs^{®} et notamment l'isododécane.

Ces huiles volatiles peuvent être présentes dans la composition en une teneur allant de 5 à 97,99 % du poids total de la composition, et mieux de 30 à 75 %.

Dans un mode particulier de réalisation de l'invention, on choisit la phase grasse liquide dans le groupe comprenant :
- les composés liquides non aqueux ayant un paramètre de solubilité global selon l'espace de solubilité de HANSEN inférieur à 17 (MPa)^{1/2},
- les monoalcools ayant un paramètre de solubilité global selon l'espace de solubilité de HANSEN inférieur ou égal à 20 (MPa)^{1/2}, et
- leurs mélanges.

La dispersion de polymère peut être fabriquée comme décrit dans le document EP-A-749 747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissout dans un solvant appelé, dans la suite de la présente description, « solvant de synthèse ». Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20 % en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30 % en poids de stabilisant par rapport au mélange initial de monomères, et de préférence 5-20 % en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut être choisi parmi les copolymères blocs greffés ou séquencés comprenant au moins un bloc de type polyorganosiloxane et au moins un polyéther. Le bloc polyorganopolysiloxane peut être notamment un polydiméthylsiloxane ou bien encore un poly alkyl (C₂-C₁₈) méthyl siloxane ; le bloc polyéther peut être un poly alkylène en C₂-C₁₈, en particulier polyoxyéthylène et/ou polyoxypropylène. En particulier, on peut utiliser les diméthicones copolyol ou encore les alkyl (C₂-C₁₈) méthicones copolyol. On peut par exemple utiliser le diméthicone copolyol vendu sous la dénomination « DOW CORNING 3225C » par la société DOW CORNING, ou le lauryl méthicone copolyol vendu sous la dénomination « DOW CORNING Q2-5200 » par la société « DOW CORNING ».

Comme polymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isopropène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont également hydrogénées. Ainsi, de façon connue, la polymérisation de l'isopropène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type « dibloc » ou tribloc » du type polystyrène/polyisoprène, polystyrène/polybutadiène tels que ceux vendus sous le nom de « LUVITOL HSB » par BASF, du type polystyrène/copoly(éthylène-propylène) tels que ceux vendus sous le nom de « KRATON » par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthylacrylate de méthyle) polyisobutylène ou les copolymères greffés à squelette poly(méthacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C₂-C₁₈, en particulier polyoxyéthylène et/ou polyoxypropylène, on peut citer les copolymères bi- ou triséquencés polyoxyéthylène/polybutadiène ou polyoxyéthylène/polyisobutylène.

On peut également employer des copolymères de (méth)acrylates d'alkyle en C₁-C₄, et de (méth)acrylates d'alkyle en C₈-C₃₀. On peut en particulier citer le copolymère méthacrylate de stéaryle/méthacrylate de méthyle.

Lorsqu'on utilise un polymère statistique en tant que stabilisant, on le choisit de manière à ce qu'il possède une quantité suffisante de groupements le rendant soluble dans le solvant de synthèse envisagé.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy (C₂-C₁₈)alkylène et notamment polyoxypropyléné et/ou oxyéthyléné.

Lorsque la phase grasse liquide ne comprend pas d'huile de silicone, l'agent stabilisant est de préférence choisi dans le groupe constitué par :
- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle en C₁-C₄, et d'acrylates ou de méthacrylates d'alkyle en C₈-C₃₀,
- (c) les copolymères blocs greffés ou séquencés comprenant au moins un bloc résultant de la polymérisation d'au moins un monomère éthylénique, à liaisons éthyléniques conjuguées, et d'au moins un bloc d'un polymère vinylique ou acrylique ou d'un polyéther ou d'un polyester, ou leurs mélanges.

De préférence, on utilise des polymères dibloc comme agent stabilisant.

Plus préférentiellement, on peut utiliser les acrylates ou méthacrylates de méthyle copolymérisés avec l'acide acrylique stabilisé en surface dans de l'isododécane par un copolymère dibloc séquencé polystyrène/copoly(éthylène-propylène).

La composition selon l'invention peut, le cas échéant, comprendre également un agent plastifiant favorisant la formation d'un film avec le polymère filmogène. Un tel agent plastifiant peut être choisi parmi tous les composés connus de l'homme du métier comme étant susceptibles de remplir la fonction recherchée.

### POLYMERE(S) SOLUBLE(S) DANS LE MILIEU SOLVANT ET AYANT AU MOINS UNE PARTIE CRISTALLISABLE

La composition selon l'invention comprend au moins un polymère soluble dans le milieu solvant, notamment non aqueux, de la composition selon l'invention et ayant au moins une partie cristallisable.

Par « polymère soluble dans le milieu solvant », on entend un polymère qui, lorsqu'il est introduit seul en une quantité en matière sèche au moins supérieure à 0,01 % en poids et en une quantité correspondante à celle envisagée pour la composition finale désirée, est soluble dans le milieu solvant à température ambiante, généralement de l'ordre de 25 °C, et sous pression atmosphérique (750 mm de Hg, soit 10⁵ Pa).

Comme précisé précédemment, ce polymère est différent du polymère filmogène retenu dans la composition selon l'invention.

Au sens de la présente invention, on désigne sous le terme de « polymère » un composé possédant au moins deux motifs de répétition, notamment au moins trois motifs de répétition, en particulier au moins dix motifs de répétition, voire au moins quinze motifs de répétition. Le polymère conforme à l'invention est généralement composé d'au moins deux motifs répétitifs de nature différente (copolymère). Les polymères utilisés dans l'invention sont généralement d'origine synthétique et sont caractérisés par des masses molaires allant de 200 à 1 000 000 g/mol, en particulier de 500 à 500 000 g/mol et plus particulièrement de 1 000 à 300 000 g/mol.

Plus précisément, les polymères utilisés dans la présente invention sont des copolymères solubilisés et non cristallisés dans le milieu à température ambiante et comportent obligatoirement au moins une partie cristallisable désignée A et au moins une partie non cristallisable dite amorphe, désignée B.

De part cette structure spécifique, ils possèdent avantageusement à la fois une affinité pour les cires grâce à la partie A et une affinité pour le solvant grâce à la partie B et participent donc à ce titre efficacement à la dispersion des cires dans le milieu solvant notamment non aqueux.

La partie cristallisable des polymères utilisés dans la présente invention représente au moins 5 %, en particulier au moins 10 % et au plus 50 %, et plus particulièrement de 30 à 50 % en poids du poids total de chaque polymère.

La partie cristallisable A d'un copolymère selon l'invention peut être figurée par une chaîne pendante liée au squelette dudit polymère et/ou une séquence intégrée directement dans ce squelette et/ou au moins une chaîne terminale. Ces copolymères peuvent être de toute structure chimique : copolymères statistiques, séquencés, greffés et/ou dendrimères.

De la même façon, la partie amorphe d'un copolymère selon l'invention peut être figurée par une chaîne pendante liée au squelette dudit copolymère et/ou une séquence intégrée directement dans ce squelette et/ou au moins une chaîne terminale.

On entend désigner au sens de l'invention par le terme ou l'expression :
- « partie cristallisable A », un enchaînement d'au moins 5 motifs de répétition et tel que si l'on prenait l'homopolymère correspondant à ce motif de répétition, il serait caractérisé par un taux de cristallinité supérieur à 30 %,
- « partie amorphe B », un enchaînement d'au moins 5 motifs de répétition et tel que si l'on prenait l'homopolymère correspondant à ce motif de répétition, il serait caractérisé par un taux de cristallinité inférieur à 5 %, voire nul,
- « séquence intégrée au squelette», un groupement d'atomes constitué par la répétition d'une unité monomère, faisant partie de la chaîne principale du polymère,
- «chaîne pendante ou groupement latéral », un groupement d'atomes figurant une ramification au niveau du squelette du polymère, et
- « chaîne terminale » un groupement d'atome situé à l'une au moins des extrémités du squelette.

### a) Les copolymères statistiques

Les copolymères statistiques sont de préférence des polymères à chaînes pendantes cristallisables qui comportent des motifs résultant de la polymérisation d'au moins deux monomères dont l'un au moins possède une chaîne latérale hydrophobe cristallisable appelée X qui peut être représenté par la formule I : 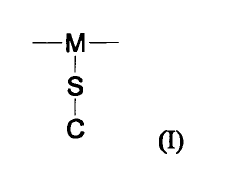 avec M représentant un atome du squelette polymérique, S représentant un espaceur, C représentant un groupe cristallisable.

Les chaînes « -S-C » cristallisables peuvent être aliphatiques ou aromatiques, linéaires, ramifiées ou cycliques, éventuellement fluorées ou perfluorées. « S » représente notamment un groupe (CH₂)ₙ ou (CH₂CH₂O)ₙ ou (CH₂O), linéaire ramifié ou cyclique, avec n étant un nombre entier allant de 0 à 22. En particulier, « S » est un groupe linéaire. En particulier, « S » et « C » sont différents.

Lorsque les chaînes « -S-C » cristallisables sont des chaînes aliphatiques hydrocarbonées, elles comportent des chaînes alkyles hydrocarbonées ayant au moins 11 atomes de carbone et au plus 40 atomes de carbone et en particulier au plus 24 atomes de carbone. Il s'agit notamment de chaînes aliphatiques ou chaînes alkyles possédant au moins 12 atomes de carbone et en particulier, il s'agit de chaînes alkyles en C₁₂-C₂₄. Lorsqu'il s'agit de chaînes alkyles fluorées ou perfluorées, elles comportent au moins 6 atomes de carbone fluorés et notamment au moins 11 atomes de carbone dont au moins 6 atomes de carbone sont fluorés.

Comme exemple de polymères à chaîne(s) pendante(s) cristallisable(s), on peut citer ceux comportant des motifs résultant de la polymérisation d'un ou plusieurs monomères suivants : les (méth)acrylates d'alkyle saturé avec le groupe alkyle en C₁₂-C₂₄, les (méth)acrylates de perfluoroalkyle avec un groupe alkyle perfluoro en C₁₂-C₁₅, les N-alkyl (méth)acrylamides avec le groupe alkyle en C₁₂ à C₂₄ avec ou sans atome de fluor, les esters vinyliques ou allyliques à chaînes alkyle ou perfluoro (alkyle) avec le groupe alkyle en C₁₂ à C₂₄ (avec au moins 6 atomes de fluor pour une chaîne perfluoro alkyle), les éthers vinyliques à chaînes alkyle ou perfluoro (alkyle) avec le groupe alkyle en C₁₂ à C₂₄ et au moins 6 atomes de fluor pour une chaîne perfluoro alkyle, les alpha-oléfines en C₁₂ à C₂₄ comme par exemple l'octadécène, les para-alkyl styrènes avec un groupe alkyle comportant de 12 à 24 atomes de carbone, leurs mélanges.

A titre illustratif de ces polymères utilisables dans la présente invention, on citera les copolymères (méth)acrylates d'alkyles linéaires et saturés en C₁₂ à C₃₀, constituant la partie cristallisable A et (méth)acrylates d'alkyles en C₄ à C₁₀ linéaires, ou en C₄ à C₃₀ ramifiés ou cycliques et/ou insaturés, constituant la partie amorphe B.

Parmi les copolymères d'esters vinyliques à groupes alkyles linéaires et saturés en C₁₂ à C₃₀ constituant la partie cristallisable A et d'esters vinyliques à groupes alkyles en C₄ à C₁₀ linéaires ou en C₄ à C₃₀ ramifiés ou cycliques et/ou insaturés constituant la partie amorphe B, on peut citer en particulier les copolymères d'acétate de vinyle et de stéarate de vinyle ou de stéarate d'allyle tel que le copolymère de stéarate d'allyle et d'acétate de vinyle vendu sous la dénomination de « Mexomère PQ^{®} » par la société CHIMEX.

Lorsque les polymères résultent d'une polycondensation, les chaînes cristallisables hydrocarbonées et/ou fluorées telles que définies ci-dessus, sont portées par un monomère qui peut être un diacide, un diol, une diamine ou un di-isocyanate.

### b) Les copolymères séquencés

Ces copolymères sont constitués d'au moins deux types de séquences de nature chimique différente dont l'une est cristallisable et constitue la partie A. Dans le cas des copolymères séquencés, l'une au moins des séquences amorphes B doit être soluble dans le milieu.

On peut citer par exemple :
- des copolymères séquencés d'oléfine ou de cyclooléfine à chaîne cristallisable comme ceux issus de la polymérisation séquencée :
   - de cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbornène, 5-benzylnorbornène, 5-vinyl norbornène, 1,4,5,8-diméthano-1,2,3,4,4a,5,8a-octahydronaphtalène, dicyclopentadiène ou de leurs mélanges,
   - avec l'éthylène, propylène, 1-butène, 3-méthyl-1-butène, 1-hexène, 4-méthyl-1-pentène, 1-octène, 1-décène, 1-éicosène ou leurs mélanges,
- les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34,117-123 (1995),
- les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer aggregates with crystalline cores : the system poly(ethylene)-poly(ethylene-propylene)" de P. Richter et al., Macromolécules, 30, 1053-1068 (1997), et
- les copolymères séquencés poly(éthylène)-b-poly(éthyléthylène) cités dans l'article général "Crystallization in block copolymers" de I.W. Hamley, Advances in Polymer Science, vol 148, 113-137 (1999).

Ces polymères peuvent présenter une unique séquence cristallisable ou une répétition de séquences cristallisables. Dans ce dernier cas, ces séquences cristallisables peuvent être de nature chimique identique ou différente.

### c) Les copolymères à séquence(s) cristallisable(s) terminale(s)

On peut citer par exemple dans cette catégorie :
- les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lesquels le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination « Uniclear 100 VG^{®} » par la Société ARIZONA CHEMICAL ; et
- les polycondensats polyesters lipophiles dont les extrémités sont estérifiées par un acide ou un alcool cristallisable constitué d'une chaîne carbonée linéaire saturée en C₁₂ à C₃₀, et en particulier, l'acide 12-polyhydroxystéarique dont au moins une des extrémités est estérifiée par l'acide stéarique, tel que par exemple le « Solsperse 21000^{®} » commercialisé par la société AVECIA.

A titre complémentaire et illustratif des copolymères selon l'invention, on peut citer en particulier les copolymères éthylène/acétate de vinyle, les copolymères éthylène/anhydride maléique, les copolymères séquencés butadiène hydrogéné/isoprène et les terpolymères éthylène/anhydride maléique/acétate de vinyle.

Selon un mode de réalisation de l'invention, le polymère soluble dans le milieu solvant, notamment non aqueux, et ayant au moins une partie cristallisable ou un mélange de tels polymères, est avantageusement présent dans la composition selon l'invention en une teneur supérieure ou égale à 0,5 % en poids par rapport au poids total de la composition.

Selon un autre mode de réalisation, le polymère soluble dans le milieu solvant, notamment non aqueux, et ayant au moins une partie cristallisable ou un mélange de tels polymères, peut être présent dans la composition selon l'invention en une proportion allant de 0,01 % à 30 %, notamment de 0,1 à 20 % et en particulier de 0,5 à 10 % en poids par rapport au poids total de la composition.

### POLYMERE A MOTIF(S) STYRENIQUE(S)

Il s'agit plus particulièrement d'homopolymères ou de copolymères blocs, greffés ou séquencés, incorporant au moins un bloc de polymère styrénique.

En particulier, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène.

Comme précisé précédemment, ce polymère à motif(s) styrénique(s) est différent du polymère filmogène retenu selon l'invention.

Selon une variante particulière de l'invention, ce polymère peut être identique à celui utilisé, à titre d'agent stabilisant, pour une dispersion non aqueuse de polymère filmogène telle que définie précédemment.

Les copolymères séquencés de type «dibloc» ou «tribloc» du type polystyrène/polyisoprène, polystyrène/polybutadiène tels que ceux commercialisés sous la dénomination de « Luvitol HSB^{®}» par la société BASF, du type polystyrène/copoly(éthylène-propylène) tels que ceux commercialisés sous la dénomination de «Kraton^{®}» par la société SHELL CHEMICAL CO ou encore du type polystyrène/copoly(éthylène-butylène) conviennent tout particulièrement à l'invention.

Ce type de copolymère à motif styrénique peut avantageusement être présent en une teneur allant de 0,01 à 30 % , notamment de 0,1 à 20 %, et en particulier de 0,5 à 10 % en poids par rapport au poids total de la composition.

A titre illustratif et non limitatif d'une composition conforme à l'invention, on peut plus particulièrement citer celles comprenant au moins
- de 20 à 50 % en poids d'un polymère filmogène notamment de type dispersion non aqueuse de particules, et en particulier le poly(méthacrylate de méthyle/acide acrylique) stabilisé en surface dans de l'isododécane par un copolymère dibloc séquencé polystyrène/copoly(éthylène-propylène)
- de 0,1 à 10 % en poids d'un polymère à motif styrénique comme par exemple un polymère de type polystyrène/copoly(éthylène-propylène) ou copolymère de stéarate d'allyle et d'acétate de vinyle, et
- de 0,1 à 10 % d'un polymère à partie cristallisable et notamment d'acide 12-polyhydroxystéarique dont au moins une extrémité est estérifiée par l'acide stéarique.

### CIRE(S)

Les compositions selon l'invention comprennent une cire ou un mélange de cires en une teneur supérieure à 3 % en poids par rapport au poids total de la composition.

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination « TA-TX2i^{®} » par la société RHEO, équipé d'un mobile en inox en forme de cylindre d'un diamètre de 2 mm en mesurant l'évolution de la force (force de compression ou force d'étirement) (F) en fonction du temps, pendant l'opération suivante :

Le mobile est déplacé à la vitesse de 0,1 mm/s puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté.

A titre illustratif des cires convenant à l'invention, on peut notamment citer les cires hydrocarbonées comme la cire d'abeilles, la cire de lanoline, les cires d'insectes de Chine, la cire de sumac, les paraffines, certaines cires de polyéthylène et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale « Iso-Jojoba-50^{®} », l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de « Hest 2T-4S^{®} » par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de « Phytowax ricin 16L64^{®} » et « 22L73^{®} » par la société SOPHIM. De telles cires sont décrites dans la demande FR-A- 2 792 190.

Dans la présente invention, on peut également utiliser des cires fournies sous forme de petites particules ayant une dimension de l'ordre de 0,5 à 30 micromètres, en particulier de 1 à 20 micromètres, et plus particulièrement de 5 à 10 micromètres, désignées par la suite par l'expression « micro cires ». A des fins de distinction, les cires mises en oeuvre selon l'invention sous forme de fragments de dimension plus élevée sont par la suite désignées par l'expression « cires de type traditionnel ».

Comme micro cires pouvant être utilisées dans les compositions selon l'invention, on peut citer notamment les micro cires de carnauba telles que celle commercialisée sous la dénomination de « MicroCare 350^{®} » par la société MICRO POWDERS, les micro cires de cire synthétique telles que celle commercialisée sous la dénomination de « MicroEase 114S^{®} » par la société MICRO POWDERS, les micro cires constituées d'un mélange de cire de carnauba et de cire de polyéthylène telles que celles commercialisées sous les dénominations de « Micro Care 300^{®} » et « 310^{®} » par la société MICRO POWDERS, les micro cires constituées d'un mélange de cire de carnauba et de cire synthétique telles que celle commercialisée sous la dénomination « Micro Care 325^{®} » par la société MICRO POWDERS, les micro cires de polyéthylène telles que celles commercialisées sous les dénominations de « Micropoly 200^{®} », « 220^{®} », « 220L^{®} » et « 250S^{®} » par la société MICRO POWDERS et les micro cires de polytétrafluoroéthylène telles que celles commercialisées sous les dénominations de «Microslip 519^{®} » et « 519 L^{®} » par la société MICRO POWDERS.

Parmi les micro cires citées précédemment, certaines telles que par exemple la micro cire de carnauba, la micro cire de cire synthétique « MicroEase 114S^{®} », ou la micro cire constituée d'un mélange de cire de carnauba et de cire synthétique « MicroCare 325^{®} » présentent un point de fusion commençante supérieur ou égal à 45 °C.

Dans la composition selon l'invention, on peut bien entendu utiliser un mélange de cires et notamment utiliser une ou plusieurs cires de type traditionnel telles que notamment une cire collante et/ou une cire à point de fusion commençante supérieur ou égal à 45 °C, et une ou plusieurs cires dites micro cires.

La composition selon l'invention contient généralement de 10 à 70 % en poids de cires. En particulier, elle peut contenir de 15 à 65 %, plus particulièrement de 20 à 60 %, voire de 25 à 55 % en poids de cire(s) par rapport au poids total de la composition.

La cire ou le mélange de cires est présent, dans les compositions selon l'invention, sous la forme d'une dispersion de particules dans le milieu solvant en particulier non aqueux.

Les particules de cire peuvent présenter des formes variées. Elles peuvent notamment être sensiblement sphériques.

Une observation au microscope d'un échantillon de la composition, à température ambiante, montre une bonne dispersion des particules de cires dans ledit milieu, avec peu ou pas d'agrégat de ces particules, voire une répartition des particules sensiblement identique dans toutes les directions.

### Matière colorante

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

### Charges

La composition selon l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination « d'Orgasol^{®} » par la société ATOCHEM, de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme celles commercialisées sous la dénomination « d'Expancel^{®} » par la société NOBEL INDUSTRIE, les poudres acryliques telles que celles commercialisées sous la dénomination « Polytrap^{®} » par la société DOW CORNING, les particules de polyméthacrylate de méthyle et les microbilles de résine de silicone (« Tospearls^{®} » de TOSHIBA, par exemple), le carbonate de calcium précipité, le carbonate et l'hydrocarbonate de magnésium, l'hydroxyapatite, les microsphères de silice creuses (« Silica Beads^{®} » de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

Les charges peuvent représenter de 0,1 à 25 %, en particulier de 1 à 20 % en poids par rapport au poids total de la composition.

La composition de l'invention peut comprendre, en outre, tout additif cosmétiquement acceptable en particulier choisi parmi ceux usuellement utilisés en cosmétique tels que les antioxydants, les conservateurs, les parfums, les neutralisants, les plastifiants, les épaississants ou gélifiants, les fibres, les actifs cosmétiques et leurs mélanges.

Les gélifiants utilisables dans les compositions selon l'invention sont généralement lipophiles et peuvent être organiques ou minéraux, polymériques ou moléculaires.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de « Bentone 38V^{®} » par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6^{ème} édition, 1995). Elles sont par exemple commercialisées sous les références « Aerosil R812^{®} » par la société DEGUSSA, « CAB-O-SIL TS-530^{®}» par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6^{ème} édition, 1995). Elles sont par exemple commercialisées sous les références « Aerosil R972^{®} », et « Aerosil R974^{®} » par la société DEGUSSA, « CAB-O-SIL TS-610^{®} » et « CAB-O-SIL TS-720^{®} » par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de «KSG6^{®}», «KSG16^{®}» et de «KSG18^{®}» par la société SHIN-ETSU, de «Trefil E-SOSC^{®}» et «Trefil E-506C^{®} » par la société DOW-CORNING, de «Gransil SR-CYC^{®}», «SR DMF10^{®}», «SR-DC556^{®}», «SR 5CYC gel^{®}», « SR DMF 10 gel^{®}» et de «SR DC 556 gel^{®}» par la société GRANT INDUSTRIES, de «SF 1204^{®}» et de «JK 113^{®}» par la société GENERAL ELECTRIC ; l'éthylcellulose comme celle vendue sous la dénomination d'«Ethocel^{®}» par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges.

Parmi les gélifiants pouvant être utilisés dans les compositions selon l'invention, on peut encore citer les esters de dextrine et d'acide gras, tels que les palmitates de dextrine, notamment tels que ceux commercialisés sous les dénominations de « Rheopearl TL^{®} » ou « Rheopearl KL^{®} » par la société CHIBA FLOUR.

La composition selon l'invention peut en outre comprendre des fibres qui permettent une amélioration de l'effet allongeant.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Comme actifs cosmétiques pouvant être utilisés dans les compositions selon l'invention, on peut citer notamment des émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La présente invention a encore pour objet un procédé de maquillage des fibres kératiniques, dans lequel on applique sur lesdites fibres kératiniques notamment les cils, une composition telle que définie précédemment.

Les compositions de l'invention peuvent être en particulier appliquées sur les cils, à l'aide d'une brosse ou d'un peigne.

Les exemples qui suivent sont présentés à titre illustratif et non limitatif de l'invention.

### Exemple

Les compositions des formulations testées figurent dans le tableau I ci-après.

**TABLEAU I**

| | **Formule comparative n° 1** | **Formule comparative n° 2** | **Formule comparative n° 3** | **F1** | **F2** |
|---|---|---|---|---|---|
| Cire de Carnauba | 4,5 | 4,7 | 4,7 | 4,7 | 4,7 |
| Cire d'abeille | 8,3 | 13,7 | 13,7 | 13,7 | 13,7 |
| Cire de polyoléfine (Performa V260 de New Phase Technologies) | --- | 0,6 | 0,6 | 0,6 | 0,6 |
| Cire de Paraffine | 2,2 | --- | --- | --- | --- |
| Polybutylène (Indopol H-1500 d'AMOCO) | --- | 3 | 3 | 3 | 3 |
| Stéarate de l'oligomère de l'acide 12-polyhydroxystéarique (Solsperse 21000 d'AVECIA) | --- | --- | 0,6 | 0,6 | 0,6 |
| Copolymère acétate de vinyle/stérarate d'allyle (Mexomère PQ de la société CHIMEX) | 2,2 | 0,75 | 0,75 | 0,75 | 0,75 |
| Polylaurate de vinyle (Mexomère PP de la société CHIMEX) | 0,75 | 0,25 | 0,25 | 0,25 | 0,25 |
| Copolymère tribloc styrène/éthylène/butylène (Kraton G-1650E de SHELL) | --- | 0,5 | --- | 0,5 | 0,5 |
| Dispersion de particules de poly(méthacrylate de méthyle/acide acrylique) stabilisées en surface dans de l'isododécane par un copolymère dibloc séquencé pllystyrène/copoly(éthylène-propylène) vendu sous le nom de KRATON G1701 à 24,5% en Matière Séche de polymères (Mexomère PAP de Chimex) | 50 | 51,5 | 51,5 | 51,5 | 25 |
| Pigments (oxydes de fer noir) | 4,6 | 3,5 | 3,5 | 3,5 | 3,5 |
| Propylène carbonate | 1,74 | --- | --- | --- | --- |
| Conservateurs | qs | qs | qs | qs | Qs |
| Isododécane | Qsp 100 | Qsp 100 | Qsp 100 | Qsp 100 | Qsp 100 |

Les pourcentages précisés sont exprimés en pourcentage en poids de matières premières considérées.

La résistance au frottement de ces formulations est présentée dans le tableau II ci-après. Celle-ci a été évaluée selon le protocole suivant :

Les éprouvettes standardisées de faux cils (constituées de cheveux caucasiens) sont maquillées selon le protocole suivant : 3 fois 10 passages de brosse avec 2 minutes de séchage entre chaque série puis séchage pendant une heure.

Les éprouvettes sont ensuite pincées avec un coton disque imbibé d'eau.

L'évaluation de la tenue consiste à compter le nombre de cotons qu'il faut pour désagréger le film de mascara. Le début de la désagrégation correspond à l'apparition de traces de mascara sur le coton.

Les résultats montrent que seules les compositions selon l'invention manifestent une tenue satisfaisante.

**TABLEAU II**

| | | Formule comparative n° 1 | Formule comparative n° 2 | Formule compara tive n° 3 | **F1** selon l'invention | **F2** selon l'invention |
|---|---|---|---|---|---|---|
| Polymère filmogène | poly(méthacrylate de méthyle/acide acrylique) stabilisées en surface dans de l'isododécane par un copolymère dibloc séquencé polystyrène/copoly(ét hylène-propylène) (Mexomère PAP de CHIMEX) | 50 | 51,5 | 51,5 | 51,5 | 25 |
| Polymère à groupement styrénique | Copolymère tribloc styrène/ethylène/butyl ène (Kraton G-1650E de SHELL) | - | 0,5 | - | 0,5 | 0,5 |
| Polymère à partie cristallisable | Stéarate de l'oligomère de l'acide 12-polyhydroxystéarique (Solsperse 21000 d'AVECIA) | - | - | 0,6 | 0,6 | 0,6 |
| **Test de frottement à l'eau** | | 7 | 9 | 7 | 20 | 20 |

## Revendications

1. Composition cosmétique de soin et/ou de maquillage de fibres kératiniques, **caractérisée en ce qu'**elle comprend dans un milieu solvant au moins :
(i) un polymère filmogène,
(ii) un polymère à motif(s) styrénique(s) distinct du composé (i), et
(iii) un polymère soluble dans ledit milieu solvant et ayant au moins une partie cristallisable et distinct du composé (i), ledit polymère étant présent en une teneur allant de 0,1 à 20 % en poids par rapport au poids total de la composition.

2. Composition selon la revendication 1, **caractérisée en ce que** le polymère filmogène est présent dans la composition sous la forme d'une dispersion de particules de polymères stabilisées en surface dans une phase grasse liquide.

3. Composition selon la revendication 1 ou 2, **caractérisée en ce que** le polymère filmogène est choisi parmi les polymères radicalaires, les polycondensats, les polymères d'origine naturelle et leurs mélanges.

4. Composition selon l'une quelconque des revendications 1 à 3, **caractérisée en ce que** le polymère filmogène est choisi parmi les polyuréthannes, polyéther-polyuréthannes, polyuréthannes-acryliques, polyesters, polyesters amides, polyesters à chaîne grasse alkyle ; polymères ou copolymères acryliques et/ou vinyliques ; polymères siliconés, polymères fluorés ; polyurées ; polyurée-polyuréthannes ; polyester-polyuréthannes ; polyacrylamides ; et leurs mélanges.

5. Composition selon l'une quelconque des revendications 1 à 4, **caractérisée en ce que** les particules de polymères sont stabilisées par un stabilisant choisi parmi les polymères séquencés, les polymères greffés, les polymères statistiques et leurs mélanges.

6. Composition selon la revendication 5, **caractérisée en ce que** le stabilisant est choisi parmi les polymères siliconés greffés avec une chaîne hydrocarbonée ; les polymères hydrocarbonés greffés avec une chaîne siliconée ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins un polyéther ; les copolymères de (méth)acrylates d'alkyle en C₁-C₄ et de (méth)acrylates d'alkyle en C₈-C₃₀; les copolymères blocs greffés ou séquencés comprenant au moins un bloc résultant de la polymérisation de monomères éthyléniques comportant une ou plusieurs liaisons éthyléniques éventuellement conjuguées et au moins un bloc de polymère styrénique ; les copolymères blocs greffés ou séquencés comprenant au moins un bloc résultant de la polymérisation de monomères éthyléniques et au moins un bloc d'un polymère acrylique ; les copolymères blocs greffés ou séquencés comprenant au moins un bloc résultant de la polymérisation de monomère éthylénique et au moins un bloc d'un polyéther.

7. Composition selon la revendication 5 ou 6, **caractérisée en ce que** l'agent stabilisant présent en surface des particules est identique au composé (ii).

8. Composition selon l'une quelconque des revendications 1 à 7, **caractérisée en ce que** le polymère filmogène est présent en une teneur en matière sèche allant de 0,1 % à 55 % en poids, en particulier de 0,5 % à 40 % en poids, et notamment de 1 % à 30 % en poids par rapport au poids total de la composition.

9. Composition selon l'une quelconque des revendications 2 à 8, **caractérisée en ce que** la phase grasse liquide est constituée d'huiles d'origine minérale, animale, végétale ou synthétique, hydrocarbonées, fluorées et/ou siliconées, seules ou en mélange.

10. Composition selon l'une quelconque des revendications 2 à 9, **caractérisée en ce que** la phase grasse liquide est choisie dans le groupe comprenant :
- les composés liquides non aqueux ayant un paramètre de solubilité global selon l'espace de solubilité de HANSEN inférieur à 17 (MPa)^{1/2},
- les monoalcools ayant un paramètre de solubilité global selon l'espace de solubilité de HANSEN inférieur ou égal à 20 (MPa)^{1/2}, et
- leur mélanges.

11. Composition selon l'une quelconque des revendications précédentes, **caractérisé en ce que** le composé (ii) est un copolymère comprenant au moins un bloc résultant de la polymérisation d'au moins un monomère éthylénique à une ou plusieurs liaisons éthyléniques éventuellement conjuguées et d'au moins un bloc d'un polymère styrénique.

12. Composition selon la revendication 11, **caractérisée en ce que** le composé est choisi parmi les copolymères de type «dibloc» ou « tribloc » du type polystyrène/polyisoprène, polystyrène/polybutadiène et polystyrène/copoly(éthylène-propylène).

13. Composition selon l'une quelconque des revendications précédentes, **caractérisée en ce que** la partie cristallisable du polymère (iii) représente au moins 5 %, en particulier au moins 10 % et au plus 50 %, et plus particulièrement de 30 à 50 % en poids du poids total dudit polymère.

14. Composition selon l'une quelconque des revendications précédentes, **caractérisée en ce que** ledit polymère (iii) est choisi parmi les copolymères de (méth)acrylates d'alkyles linéaires et saturés en C₁₂ à C₃₀ et de (méth)acrylates d'alkyles en C₄ à C₁₀ linéaires ou en C₄ à C₃₀ ramifiés ou cycliques et/ou insaturés, les copolymères d'esters vinyliques à groupes alkyles linéaires et saturés en C₁₂ à C₃₀ et d'esters vinyliques à groupes alkyles en C₄ à C₁₀ linéaires, ou en C₄ à C₃₀ ramifiés ou cycliques et/ou insaturés, les polycondensats de type polyamide résultant de la condensation entre (α) au moins un acide choisi parmi les acides dicarboxyliques comprenant au moins 32 atomes de carbone et (β) un alkylène diamine, ledit polycondensat comprenant au moins un groupe acide carboxylique terminal estérifié ou amidifié avec au moins un mono alcool ou un mono amine comprenant de 12 à 30 atomes de carbone linéaires et saturés et les polycondensats polyesters lipophiles dont les extrémités sont estérifiées par un acide ou un alcool cristallisable constitué d'une chaîne carbonée linéaire saturée en C₁₂ à C₃₀.

15. Composition selon l'une quelconque des revendications précédentes, **caractérisée en ce que** ledit polymère (iii) est choisi parmi les copolymères acétate de vinyle/stéarate de vinyle, acétate de vinyle/stéarate d'allyle acétate de vinyle/éthylène, éthylène anhydride maléique, les copolymères séquencés butadiène hydrogénés/isoprène et l'acide 12-polyhydroxystéarique dont au moins une des extrémités est estérifiée par l'acide stéarique.

16. Composition selon l'une quelconque des revendications précédentes, **caractérisée en ce que** ledit polymère (ii) est présent en une teneur allant de 0,01 à 30 % , notamment de 0,1 à 20 %, et en particulier de 0,5 à 10 % en poids par rapport au poids total de la composition.

17. Composition selon l'une quelconque des revendications précédentes, **caractérisée en ce que** ledit polymère (iii) est présent en une teneur allant de 0,5 à 10 % en poids par rapport au poids total de la composition.

18. Composition selon l'une quelconque des revendications précédentes, **caractérisée en ce qu'**elle comprend au moins
- 20 à 50 % en poids d'au moins un polymère filmogène,
- 0,1 à 10 % en poids d'au moins un polymère à motif styrénique, et
- 0,1 à 10 % en poids d'au moins un polymère à partie cristallisable.

19. Composition selon l'une quelconque des revendications précédentes, **caractérisée en ce que** ledit milieu solvant est un milieu non aqueux contenant au moins un composé volatil non soluble dans l'eau et liquide à température ambiante.

20. Composition selon la revendication 19, **caractérisée en ce que** le composé volatil non soluble dans l'eau est choisi parmi les huiles hydrocarbonées, siliconées et/ou fluorées, les solvants organiques et leurs mélanges, et en particulier parmi les huiles hydrocarbonées ayant de 8 à 16 atomes de carbone et leurs mélanges.

21. Composition selon la revendication 19 ou 20, **caractérisée en ce que** ledit composé volatil non soluble dans l'eau est présent dans la composition en une teneur allant de 5 à 55 %, notamment de 10 à 50 % et en particulier de 20 à 45 % en poids par rapport au poids total de la composition.

22. Composition selon l'une quelconque des revendications précédentes, **caractérisée en ce que** sa teneur en eau et/ou solvant(s) hydrosoluble(s) est supérieure ou égale à 0,5 %, notamment allant de 1 à 18 %, et en particulier de 2 à 15 % en poids par rapport au poids total de la composition.

23. Composition selon l'une quelconque des revendications précédentes, **caractérisée en ce qu'**elle comprend en outre au moins une cire.

24. Composition selon la revendication 23, **caractérisée en ce que** la cire est choisie parmi les cires, solides et rigides à température ambiante ayant un point de fusion supérieur ou égal à 30 °C, en particulier supérieur ou égal à 45 °C, notamment supérieur ou égal à 55 °C.

25. Composition selon la revendications 23 ou 24, **caractérisée en ce que** la cire est choisie parmi les cires hydrocarbonées comme la cire d'abeille, la cire de lanoline, les cires d'insectes de Chine, la cire de sumac, les paraffines, les cires de polyéthylène, les copolymères cireux ainsi que leurs esters ; les cires obtenues par hydrogénation catalytique d'huiles animales ou végétales ayant des chaînes grasses, linéaires ou ramifiées en C₈-C₃₂ comme l'huile de jojoba partiellement hydrogénée isomérisée trans, l'huile de tournesol hydrogénée, l'huile de ricin hydrogénée, l'huile de coprah hydrogénée, l'huile de lanoline hydrogénée et le tétrastéarate de di-(triméthylol-1,1,1 propane) et les cires obtenues par hydrogénation d'huile de ricin estérifiée avec l'alcool cétylique.

26. Composition selon la revendication 23, **caractérisée en ce que** ladite cire est choisie parmi les (hydroxystéaryloxy) stéarate d'alkyle en C₂₀-C₄₀.

27. Composition selon l'une quelconque des revendications 23 à 26, **caractérisée en ce que** la teneur totale en cire est de 10 à 70 %, notamment de 15 à 65 %, en particulier de 20 à 60 %, et plus particulièrement de 25 à 55 % en poids par rapport au poids total de la composition.

28. Composition selon l'une quelconque des revendications précédentes, **caractérisée en ce qu'**elle comprend en outre au moins une matière colorante.

29. Composition selon l'une quelconque des revendications précédentes, **caractérisée en ce qu'**elle comprend en outre au moins une charge.

30. Composition selon l'une quelconque des revendications précédentes, **caractérisée en ce qu'**elle comprend en outre au moins un additif cosmétiquement acceptable choisi parmi les antioxydants, les conservateurs, les parfums, les neutralisants, les plastifiants, les fibres, les gélifiants, les actifs cosmétiques et leurs mélanges.

31. Procédé de maquillage des fibres kératiniques, **caractérisé en ce que** l'on applique sur lesdites fibres kératiniques notamment les cils, une composition telle que définie dans l'une quelconque des revendications 1 à 30.

## Claims

1. Cosmetic composition for the care and/or make-up of keratin fibres, **characterized in that** it comprises at least the following in a solvent medium:
(i) a film-forming polymer,
(ii) a polymer with one or more styrene units that is different from compound (i), and
(iii) a polymer that is soluble in said solvent medium, has at least one crystallizable part and is different from compound (i) said polymer being present in an amount ranging from 0.1 to 20 % by weight, based on the total weight of the composition.

2. Composition according to Claim 1, **characterized in that** the film-forming polymer is present in the composition in the form of a dispersion of polymer particles stabilized on the surface in a liquid fatty phase.

3. Composition according to Claim 1 or 2, **characterized in that** the film-forming polymer is selected from free radical polymers, polycondensates, polymers of natural origin, and mixtures thereof.

4. Composition according to any one of Claims 1 to 3, **characterized in that** the film-forming polymer is selected from polyurethanes, polyetherpolyurethanes, acrylic polyurethanes, polyesters, polyesteramides, polyesters with an alkyl fatty chain, acrylic and/or vinylic polymers or copolymers, silicone polymers, fluorinated polymers and mixtures thereof.

5. Composition according to any one of Claims 1 to 4, **characterized in that** the polymer particles are stabilized with a stabilizer selected from sequence polymers, graft polymers, random polymers and mixtures thereof.

6. Composition according to Claim 5, **characterized in that** the stabilizer is selected from silicone polymers grafted with a hydrocarbon chain; hydrocarbon polymers grafted with a silicone chain; graft or sequence block copolymers comprising at least one block of the polyorganosiloxane type and at least one block of a free radical polymer; graft or sequence block copolymers comprising at least one block of the polyorganosiloxane type and at least one polyether; copolymers of C₁-C₄-alkyl (meth)acrylates and C₈-C₃₀-alkyl (meth)acrylates; graft or sequence block copolymers comprising at least one block resulting from the polymerization of ethylenic monomers having one or more optionally conjugated double bonds, and at least one block of a styrene polymer; graft or sequence block copolymers comprising at least one block resulting from the polymerization of ethylenic monomers, and at least one block of an acrylic polymer; and graft or sequence block copolymers comprising at least one block resulting from the polymerization of an ethylenic monomer, and at least one block of a polyether.

7. Composition according to Claim 5 or 6, **characterized in that** the stabilizer present on the surface of the particles is identical to compound (ii).

8. Composition according to any one of Claims 1 to 7, **characterized in that** the film-forming polymer is present in a solids content ranging from 0.1% to 55% by weight, particularly from 0.5% to 40% by weight and especially from 1% to 30% by weight, based on the total weight of the composition.

9. Composition according to any one of Claims 2 to 8, **characterized in that** the liquid fatty phase consists of hydrocarbon, fluorinated and/or silicone oils of mineral, animal, vegetable or synthetic origin, by themselves or in a mixture.

10. Composition according to any one of Claims 2 to 9, **characterized in that** the liquid fatty phase is selected from the group comprising:
- non-aqueous liquid compounds having a global solubility parameter according to the HANSEN solubility space that is less than 17 (MPa)^{1/2},
- monoalcohols having a global solubility parameter according to the HANSEN solubility space that is less than or equal to 20 (MPa)^{1/2}, and
- mixtures thereof.

11. Composition according to any one of the preceding claims, **characterized in that** compound (ii) is a copolymer comprising at least one block resulting from the polymerization of at least one ethylenic monomer having one or more optionally conjugated double bonds, and at least one block of a styrene polymer.

12. Composition according to Claim 11, **characterized in that** the compound is selected from "diblock" or "triblock" copolymers of the polystyrene/ polyisoprene, polystyrene/polybutadiene and polystyrene/copoly(ethylenepropylene) type.

13. Composition according to any one of the preceding claims, **characterized in that** the crystallizable part of polymer (iii) represents at least 5%, particularly at least 10% and at most 50% and more particularly from 30 to 50% by weight, based on the total weight of said polymer.

14. Composition according to any one of the preceding claims, **characterized in that** said polymer (iii) is selected from copolymers of saturated linear C₁₂ to C₃₀ alkyl (meth)acrylates and linear C₄ to C₁₀ or branched or cyclic and/or unsaturated C₄ to C₃₀ alkyl (meth)acrylates, copolymers of vinyl esters with saturated linear C₁₂ to C₃₀ alkyl groups and vinyl esters with linear C₄ to C₁₀ or branched or cyclic and/or unsaturated C₄ to C₃₀ alkyl groups, polycondensates of the polyamide type resulting from condensation between (α) at least one acid selected from dicarboxylic acids having at least 32 carbon atoms, and (β) an alkylenediamine, said polycondensate comprising at least one terminal carboxylic acid group esterified or amidified with at least one monoalcohol or monoamine having from 12 to 30 saturated linear carbon atoms, and lipophilic polyester polycondensates whose ends are esterified with a crystallizable acid or alcohol consisting of a saturated linear C₁₂ to C₃₀ carbon chain.

15. Composition according to any one of the preceding claims, **characterized in that** said polymer (iii) is selected from vinyl acetate/vinyl stearate, vinyl acetate/allyl stearate, vinyl acetate/ethylene and ethylene/maleic anhydride copolymers, hydrogenated butadiene/isoprene sequence copolymers, and poly(12-hydroxystearic acid) of which at least one end is esterified with stearic acid.

16. Composition according to any one of the preceding claims, **characterized in that** said polymer (ii) is present in an amount ranging from 0.01 to 30%, especially from 0.1 to 20% and particularly from 0.5 to 10% by weight, based on the total weight of the composition.

17. Composition according to any one of the preceding claims, **characterized in that** said polymer (iii) is present in an amount ranging from 0.5 to 10% by weight, based on the total weight of the composition.

18. Composition according to any one of the preceding claims, **characterized in that** it comprises at least:
- 20 to 50% by weight of at least one film-forming polymer,
- 0.1 to 10% by weight of at least one polymer with styrene units, and
- 0.1 to 10% by weight of at least one polymer with a crystallizable part.

19. Composition according to any one of the preceding claims, **characterized in that** said solvent medium is a non-aqueous medium containing at least one volatile, water-insoluble compound that is liquid at room temperature.

20. Composition according to Claim 19, **characterized in that** the volatile, water-insoluble compound is selected from hydrocarbon, silicone and/or fluorinated oils, organic solvents and mixtures thereof, and particularly from hydrocarbon oils having from 8 to 16 carbon atoms, and mixtures thereof.

21. Composition according to Claim 19 or 20, **characterized in that** said volatile, water-insoluble compound is present in the composition in an amount ranging from 5 to 55%, especially from 10 to 50% and particularly from 20 to 45% by weight, based on the total weight of the composition.

22. Composition according to any one of the preceding claims, **characterized in that** its content of water and/or water-soluble solvent(s) is greater than or equal to 0.5% and ranges especially from 1 to 18% and particularly from 2 to 15% by weight, based on the total weight of the composition.

23. Composition according to any one of the preceding claims, **characterized in that** it also comprises at least one wax.

24. Composition according to Claim 23, **characterized in that** the wax is selected from waxes that are solid and rigid at room temperature and have a melting point greater than or equal to 30°C, particularly greater than or equal to 45°C and especially greater than or equal to 55°C.

25. Composition according to Claim 23 or 24, **characterized in that** the wax is selected from hydrocarbon waxes such as beeswax, lanolin wax, Chinese waxes, sumac wax, paraffins, polyethylene waxes and waxy copolymers, as well as their esters; waxes obtained by the catalytic hydrogenation of animal or vegetable oils having linear or branched C₈-C₃₂ fatty chains, such as trans-isomerized, partially hydrogenated jojoba oil, hydrogenated sunflower oil, hydrogenated castor oil, hydrogenated copra oil, hydrogenated lanolin oil and di(1,1,1-trimethylolpropane) tetrastearate; and waxes obtained by the hydrogenation of castor oil esterified with cetyl alcohol.

26. Composition according to Claim 23, **characterized in that** said wax is selected from C₂₀-C₄₀-alkyl hydroxystearyloxystearates.

27. Composition according to any one of Claims 23 to 26, **characterized in that** the total wax content is 10 to 70%, especially 15 to 65%, particularly 20 to 60% and more particularly 25 to 55% by weight, based on the total weight of the composition.

28. Composition according to any one of the preceding claims, **characterized in that** it also comprises at least one colourant.

29. Composition according to any one of the preceding claims, **characterized in that** it also comprises at least one filler.

30. Composition according to any one of the preceding claims, **characterized in that** it also comprises at least one cosmetically acceptable additive selected from antioxidants, preservatives, perfumes, neutralizers, plasticizers, fibres, gelling agents, cosmetic active ingredients and mixtures thereof.

31. Method of making up keratin fibres, **characterized in that** a composition as defined in any one of Claims 1 to 30 is applied to said keratin fibres, especially eyelashes.

## Patentansprüche

1. Kosmetische Pflege- und/oder Schminkzusammensetzung von Keratinfasern, **dadurch gekennzeichnet, dass** sie in einem Lösungsmittelmedium mindestens folgendes umfasst:
(i) ein filmbildendes Polymer,
(ii) ein Polymer mit von der Verbindung (i) verschiedenem(n) styrolischem(n) Motiv(en), und
(iii) ein Polymer, das in dem Lösungsmittelmedium löslich ist und mit mindestens einem kristallisierbaren und von der Verbindung (i) verschiedenen Teil, wobei das Polymer in einem Gehalt vorhanden ist, der von 0,1 bis 20 Gew.-%, bezogen auf das Gesamtgewicht der Zusammensetzung, reicht.

2. Zusammensetzung nach Anspruch 1, **dadurch gekennzeichnet, dass** das filmbildende Polymer in der Zusammensetzung in der Form einer Dispersion von Teilchen von in einer flüssigen Fettphase oberflächenstabilisierten Polymeren vorhanden ist.

3. Zusammensetzung nach Anspruch 1 oder 2, **dadurch gekennzeichnet, dass** das filmbildende Polymer aus Radikal-Polymeren, Polykondensaten, Polymeren natürlichen Ursprungs und ihren Gemischen ausgewählt ist.

4. Zusammensetzung nach einem der Ansprüche 1 bis 3, **dadurch gekennzeichnet, dass** das filmbildende Polymer aus Polyurethanen, Polyether-Polyurethanen, Acrylpolyurethanen, Polyestern, Polyesteramiden, Polyestern mit Alkylfettsäureketten; Acryl- und/oder Vinyl-Polymeren oder -Copolymeren; Silikonpolymeren, fluorierten Polymeren, Polyharnstoffen, Polyharnstoffpolyurethanen; Polyesterpolyurethanen; Polyacrylamiden und ihren Gemischen ausgewählt ist.

5. Zusammensetzung nach einem der Ansprüche 1 bis 4, **dadurch gekennzeichnet, dass** die Teilchen von Polymeren durch ein stabilisierendes Mittel stabilisiert sind, das aus Sequenzpolymeren, Pfropfpolymeren, statistischen Polymeren ihren Gemischen ausgewählt ist.

6. Zusammensetzung Anspruch 5, **dadurch gekennzeichnet, dass** das stabilisierende Mittel ausgewählt ist aus mit einer Kohlenwasserstoffkette gepfropften Silikonpolymeren; mit einer Silikonkette gepfropften Kohlenwasserstoffpolymeren; Pfropf- oder Sequenz-Blockcopolymeren, die mindestens einen Block vom Typ eines Polyorganosiloxans und mindestens einen Block vom Typ eines radikalischen Polymers umfassen; Pfropf- oder Sequenz-Blockcopolymeren, die mindestens einen Block vom Typ eines Polyorganosiloxans und mindestens einen Polyether umfassen; Copolymeren von C₁-C₄-Alkyl(meth)acrylaten und von C₈-C₃₀-Alkyl(meth)acrylaten; Pfropf- oder Sequenz-Blockcopolymeren, die mindestens einen Block umfassen, der das Ergebnis der Polymerisation von ethylenischen Monomeren ist, die eine oder mehrere ethylenische, gegebenenfalls konjugierte Bindungen und mindestens ein Styrol-Blockpolymer umfassen; Pfropf- oder Sequenz-Blockcopolymeren, die mindestens einen Block umfassen, der das Ergebnis der Polymerisation von ethylenischen Monomeren und mindestens einem Block eines acrylischen Polymers ist; Pfropf- oder Sequenz-Blockcopolymeren, die mindestens einen Block umfassen, der das Ergebnis der Polymerisation von einem ethylenischen Monomer und mindestens einem Block eines Polyethers ist.

7. Zusammensetzung nach Anspruch 5 oder 6, **dadurch gekennzeichnet, dass** das stabilisierende Mittel, das an der Oberfläche der Teilchen vorhanden ist, mit der Verbindung (ii) identisch ist.

8. Zusammensetzung nach einem der Ansprüchel bis 7, **dadurch gekennzeichnet, dass** das filmbildende Polymer in einem Trockenmasse-Gehalt vorhanden ist, der von 0,1 bis 55 Gew.-% und insbesondere von 0,5 bis 40 Gew.-% und besonders von 1 bis 30 Gew.-%, bezogen auf das Gesamtgewicht der Zusammensetzung, reicht.

9. Zusammensetzung nach einem der Ansprüche 2 bis 8, **dadurch gekennzeichnet, dass** die flüssige Fettphase aus Ölen mineralischen, tierischen, pflanzlichen oder synthetischen Ursprungs, hydrocarbonierten, fluorierten und/oder silikonierten, allein oder im Gemisch, besteht.

10. Zusammensetzung nach einem der Ansprüche 2 bis 9, **dadurch gekennzeichnet, dass** die flüssige Fettphase aus der Gruppe ausgewählt ist, die folgendes einschließt:
- nichtwässrige flüssige Verbindungen mit einem globalen Löslichkeitsparameter gemäß dem Löslichkeitsraum von HANSEN von kleiner als 17 (MPa)^{1/2},
- Monoalkohole mit einem globalen Löslichkeitsparameter gemäß dem Löslichkeitsraum von HANSEN von kleiner oder gleich 20 (MPa)^{1/2}, und
- ihren Gemischen.

11. Zusammensetzung nach einem der vorhergehenden Ansprüche, **dadurch gekennzeichnet, dass** die Verbindung (ii) ein Copolymer ist, das mindestens einen Block umfasst, der das Ergebnis der Polymerisation von mindestens einem ethylenischen Monomer mit einer oder mehreren ethylenischen, gegebenenfalls konjugierten Bindungen und von mindestens einem Block eines Styrol-Polymers ist.

12. Zusammensetzung nach Anspruch 11, **dadurch gekennzeichnet, dass** die Verbindung aus den Copolymeren vom Typ eines "Zweler-" oder "Dreierblocks" des Typs Polystyrol/Polyisopren, Polystyrol/Polybutadien und Polystyrol/Copoly(ethylen-propylen) ausgewählt ist.

13. Zusammensetzung nach einem der vorhergehenden Ansprüche, **dadurch gekennzeichnet, dass** der kristallisierbare Teil des Polymers (iii) mindestens 5 %, insbesondere mindestens 10 % und höchstens 50 % und insbesondere 30 bis 50 Gew.-% des Gesamtgewichts des Polymers repräsentiert.

14. Zusammensetzung nach einem der vorhergehenden Ansprüche, **dadurch gekennzeichnet, dass** das Polymer (III) ausgewählt ist aus Copolymeren von (Meth)acrylaten von linearen und gesättigten C₁₂- bis C₃₀-Alkylen und von (Meth)acrylaten von linearen C₄-bis C₁₀- oder von verzweigten oder cyclischen und/oder ungesättigten C₄- bis C₃₀-Alkylen, Copolymeren von Vinylestern mit linearen und gesättigten C₁₂- bis C₃₀-Alkylgruppen und von Vinylestern mit linearen C₄- bis C₁₀-Alkylgruppen oder mit verzweigten oder cyclischen und/oder ungesättigten C₄- bis C₃₀-Alkylgruppen, Polykondensaten von Typ eines Polyamids, die das Ergebnis der Kondensation sind zwischen (α) mindestens einer Säure, die aus den Dicarbonsäuren ausgewählt ist, die mindestens 32 Kohlenstoffatome umfassen, und (β) einem Alkylendiamin, wobei das Polykondensat mindestens eine terminale Carbonsäuregruppe, die mit mindestens einem Monoalkohol oder einem Monoamin verestert oder amidiert ist, das 12 bis 30 lineare und gesättigte Kohlenstoffatome umfasst, und lipophilen Polyester-Polykondensaten, deren Enden mit einer kristallisierbaren Säure oder einem kristallisierbaren Alkohol bestehend aus einer linearen gesättigten C₁₂- bis C₃₀-Kohlenstoffkette verestert sind.

15. Zusammensetzung nach einem der vorhergehenden Ansprüche, **dadurch gekennzeichnet, dass** das Polymer (iii) ausgewählt ist aus den Copolymeren Vinylacetat/Vinylstearat, Vinylacetat/Allylstearat, Vinylacetat/Ethylen, Ethylenmaleinsäureanhydrid, den hydrierten Sequenzcopolymeren von Butadien/Isopren und 12-Polyhydroxystearinsäure, wovon mindestens eines der Enden mit Stearinsäure verestert ist.

16. Zusammensetzung nach einem der vorhergehenden Ansprüche, **dadurch gekennzeichnet, dass** das Polymer (ii) in einem Gehalt vorhanden ist, der von 0,01 bis 30 Gew.-%, insbesondere von 0,1 bis 20 Gew.-% und besonders von 0,5 bis 10 Gew.-%, bezogen auf das Gesamtgewicht der Zusammensetzung, reicht.

17. Zusammensetzung nach einem der vorhergehenden Ansprüche, **dadurch gekennzeichnet, dass** das Polymer (iii) in einem Gehalt vorhanden ist, der von 0,5 bis 10 Gew.-% bezogen auf das Gesamtgewicht der Zusammensetzung reicht.

18. Zusammensetzung nach einem der vorhergehenden Ansprüche, **dadurch gekennzeichnet, dass** sie mindestens
- 20 bis 50 Gew.-% von mindestens einem filmbildenden Polymer,
- 0,1 bis 10 Gew.-% von mindestens einem Polymer mit styrolischem Motiv, und
- 0,1 bis 10 Gew.-% von mindestens einem Polymer mit kristallisierbarem Teil umfasst.

19. Zusammensetzung nach einem der vorhergehenden Ansprüche, **dadurch gekennzeichnet, dass** das Lösungsmittelmedium ein nichtwässriges Medium ist, das mindestens eine flüchtige, nicht in Wasser lösliche und bei Umgebungstemperatur flüssige Verbindung enthält.

20. Zusammensetzung nach Anspruch 19, **dadurch gekennzeichnet, dass** die flüchtige, nicht in Wasser lösliche Verbindung aus Kohlenwasserstoffölen, silikonierten und/oder fluorierten, aus organischen Lösungsmitteln und ihren Gemischen und insbesondere aus Kohlenwasserstoffölen mit 8 bis 16 Kohlenstoffatomen und ihren Gemischen ausgewählt ist.

21. Zusammensetzung nach Anspruch 19 oder 20, **dadurch gekennzeichnet, dass** die flüchtige, nicht in Wasser lösliche Verbindung in der Zusammensetzung in einem Gehalt von 5 bis 55 Gew.-%, insbesondere von 10 bis 50 Gew.-% und besonders von 20 bis 45 Gew.-%, bezogen auf das Gesamtgewicht der Zusammensetzung, vorhanden ist.

22. Zusammensetzung nach einem der vorhergehenden Ansprüche, **dadurch gekennzeichnet, dass** ihr Gehalt an Wasser und/oder wasserlöslichem(n) Lösungsmittel(n) größer oder gleich 0,5 Gew.-% ist, insbesondere von 1 bis 18 Gew.-% und besonders von 2 bis 15 Gew.-%, bezogen auf das Gesamtgewicht der Zusammensetzung, reicht.

23. Zusammensetzung nach einem der vorhergehenden Ansprüche, **dadurch gekennzeichnet, dass** sie ferner mindestens ein Wachs umfasst.

24. Zusammensetzung nach Anspruch 23, **dadurch gekennzeichnet, dass** das Wachs ausgewählt ist aus bei Umgebungstemperatur festen und starren Wachsen mit einem Schmelzpunkt von größer oder gleich 30 °C, insbesondere von größer oder gleich 45 °C, besonders von größer oder gleich 55 °.

25. Zusammensetzung nach den Ansprüchen 23 oder 24, **dadurch gekennzeichnet, dass** das Wachs ausgewählt ist Kohlenwasserstoff-Wachsen wie Bienenwachs, Lanolin-Wachs, chinesischen Insektenwachsen, Sumac-Wachs, Paraffinen, Polyethylenwachsen, wachsartigen Copolymeren sowie ihren Estern; Wachsen, die durch katalytische Hydrierung von tierischen oder pflanzlichen Ölen mit linearen oder verzweigten C₈- bis C₃₂-Fettsäureketten erhalten werden, wie teilweise hydriertes trans-isomerisiertes Jojobaöl, hydriertes Sonnenblumenöl, hydriertes Rizinussöl, hydriertes Copraöl, hydriertes Lanolinöl und Di-(trimethylol-1,1,1-propane)-tetrastearat, und aus Wachsen, die durch Hydrierung von mit Cetylalkohol verestertem Rizinussöl erhalten werden.

26. Zusammensetzung nach Anspruch 23, **dadurch gekennzeichnet, dass** das Wachs aus (Hydroxystearoyloxy)-C₂₀-C₄₀-alkylstearat ausgewählt ist.

27. Zusammensetzung nach einem der Ansprüche 23 bis 26, **dadurch gekennzeichnet, dass** der Gesamtgehalt an Wachs von 10 bis 70 Gew.-%, insbesondere von 15 bis 65 Gew.-%, besonders von 20 bis 60 Gew.-%, ganz besonders von 25 bis 55 Gew.-% beträgt, bezogen auf das Gesamtgewicht der Zusammensetzung.

28. Zusammensetzung nach einem der vorhergehenden Ansprüche, **dadurch gekennzeichnet, dass** sie weiterhin mindestens einen Farbstoff einschließt.

29. Zusammensetzung nach einem der vorhergehenden Ansprüche, **dadurch gekennzeichnet, dass** sie weiterhin mindestens einen Füllstoff einschließt.

30. Zusammensetzung nach einem der vorhergehenden Ansprüche, **dadurch gekennzeichnet, dass** sie weiterhin mindestens einen kosmetisch verträglichen Hilfsstoff einschließt, der ausgewählt ist aus Antioxidantien, Konservierungsstoffen, Duftstoffen, Neutralisationsmitteln, Weichmachern, Fasern, Gelbildnern, kosmetischen Wirkstoffen und ihren Gemischen.

31. Verfahren zum Schminken von keratinischen Fasern, **dadurch gekennzeichnet, dass** auf die keratinischen Fasern, insbesondere die Wimpern, eine Zusammensetzung wie nach einem der Ansprüche 1 bis 30 definiert aufgebracht wird.
